# EUROPEAN PATENT SPECIFICATION

(11) **EP 2 723 241 B1**
(45) Date of publication and mention of the grant of the patent: **19.11.2014**
(21) Application number: 12738612.6
(22) Date of filing: 21.06.2012
(51) Int. Cl.: A61B 8/00, A61B 8/08, G06T 7/00, G06T 3/00

(54) **ULTRASOUND-IMAGE-GUIDE SYSTEM AND VOLUME-MOTION-BASE CALIBRATION METHOD**
ULTRASCHALL-BILDFÜHRUNGSSYSTEM UND VERFAHREN ZUR KALIBRIERUNG EINER VOLUMENBEWEGUNGSBASIS
SYSTÈME GUIDÉ PAR IMAGE ULTRASONORE ET PROCÉDÉ D'ÉTALONNAGE À BASE DE MOUVEMENT DE VOLUME

(30) Priority: 27.06.2011 US 201161501271 P
(43) Date of publication of application: 30.04.2014
(73) Proprietor: Koninklijke Philips N.V., 5656 AE Eindhoven (NL)
(72) Inventor: JAIN, Ameet Kumar, NL-5656 AE Eindhoven (NL); STANTON, Douglas Allen, NL-5656 AE Eindhoven (NL); HALL, Christopher Stephen, NL-5656 AE Eindhoven (NL)
(74) Representative: Steffen, Thomas
(86) International application number: PCT/IB2012/053138
(87) International publication number: WO 2013/001424

(56) References cited:
- EP-A1- 1 932 477
- WO-A1-2009/063360
- US-B1- 6 338 716

## Description

### FIELD OF THE INVENTION

The present invention relates to an ultrasound-image-guided system comprising one or more ultrasound probes operable to generate image volumes of an anatomical object. The present invention further relates to a volume-motion-based calibration method for operating such ultrasound-image-guided system, and a computer program implementing such method.

### BACKGROUND OF THE INVENTION

Ultrasound has in the past few decades started to become the modality of preference for interventional procedures, for example for minimally invasive interventions. A specific example is intra-procedural beating heart surgery and therapy. In particular, ultrasound-image-guided interventions are of very strong interest, for example ranging from valve placements to biopsies to ablation. Ultrasound images can here help the surgeon or therapist to navigate or guide a clinical instrument, such as a needle or a catheter for example.

One of the main limitations of these ultrasound-image-guided (navigation) systems is the requirement of a pre-calibrated ultrasound probe, wherein a position sensor for tracking needs to be attached to the ultrasound probe and a calibration of the system / ultrasound probe has to be performed, more particularly a calibration between the images of the ultrasound probe and the position sensor. It has shown that this calibration determines the performance of the whole system, making the position sensor integration both challenging and expensive. It requires an expensive pre-calibration protocol and also factory manufacturing of the system is expensive.

For example, US 2010/0081920 A1 discloses an electromagnetic (EM) tracking system for use in ultrasound and other imaging modality guided medical procedures. The system includes a tool set of various components to which electromagnetic (EM) sensors can be releasably secured. The tool set comprises an EM-trackable trochar, an EM sensor-equipped bracket, a slotted needle guide, an EM sensor-equipped adapter, and an external skin marker. However, this system is complex and requires a special pre-calibration. This yields a quite expensive system.

Document WO2009063360 discloses an ultrasound imaging system adapted to perform self-calibration based on information from a tracking system and volume-motion based information.

Document EP1932477 discloses an ultrasound imaging system comprising a positioning system which can be attached to the probe for one use event.

### SUMMARY OF THE INVENTION

It is an object of the present invention to provide an ultrasound-image-guided system that is less expensive, but still provides reliable results needed for medical interventions. It is a further object to provide a volume-motion-based calibration method for operating such system and a computer program implementing such method.

In a first aspect of the present invention an ultrasound-image-guided system is presented that comprises one or more ultrasound probes operable to generate image volumes of an anatomical object, and an adapter device comprising at least one position sensor. The adapter device is, for one use event, attachable to one of the ultrasound probes, wherein the at least one position sensor is at a variable position with respect to the one or more ultrasound probes from one use event to another use event. The system further comprises a tracking device operable to generate tracking data representative of a tracking of the at least one position sensor within a coordinate system, and an ultrasound imaging device operable to generate imaging data of the anatomical object based on the image volumes. The system further comprises a computation device operable to automatically self-calibrate, for each use event, the imaging data with respect to the coordinate system of the at least one position sensor by calculating a calibration matrix using an image based volume motion and a tracking based volume motion. The image based volume motion represents an image motion of at least two image volumes derived from the imaging data. The tracking based volume motion represents a tracking motion of the image volumes derived from the tracking data.

In a further aspect of the present invention a volume-motion-based calibration method for operating an ultrasound-image-guided system is presented comprising one or more ultrasound probes operable to generate image volumes of an anatomical object, and an adapter device comprising at least one position sensor. The adapter device is, for one use event, attachable to one of the ultrasound probes. The at least one position sensor is at a variable position with respect to the one or more ultrasound probes from one use event to another use event. The method comprises the steps of a) generating tracking data representative of a tracking of the at least one position sensor within a coordinate system; b) generating imaging data of the anatomical object based on the image volumes; and c) automatically self-calibrating, for each use event, the imaging data with respect to the coordinate system of the at least one position sensor by calculating a calibration matrix using an image based volume motion and a tracking based volume motion. The image based volume motion represents an image motion of at least two image volumes within the coordinate system derived from the imaging data. The tracking based volume motion represents a tracking motion of the image volumes within the coordinate system derived from the tracking data.

In a further aspect of the present invention a computer program is presented comprising code means for causing a computer to carry out the steps of the method disclosed herein when said computer program is carried out on the computer.

The basic idea of the invention is to use an imprecise adapter device in combination with a specific automatic self-calibration method for calculating a calibration matrix. An (uncalibrated) system or ultrasound probe is provided, having an adapter device with position (tracking) sensor(s) attachable or attached to the ultrasound probe, wherein the adapter device can be imprecisely manufactured. Thus, there is no need to provide a specially manufactured adapter device for a specific ultrasound probe. The adapter device can in particular fit to multiple different ultrasound probes (or types of ultrasound probes). In this way, a more plug-and-play mechanism (adapter device) is presented, that is significantly cheaper. Thus, the adapter device can be mass-manufactured, for example using a casting or rapid prototyping/printing technique that offers micron grade repeatability. In particular, the adapter device can be removably attachable or attached to the ultrasound probe. In particular, the adapter device and/or ultrasound probe can be adapted for in-vivo application or use.

The imprecisely manufactured adapter device comprising the position sensor(s) is, for one use event, attachable or attached to one of the ultrasound probes. A use event refers to the attachment of the adapter device to one of the ultrasound probes and the use of this adapter-probe combination (for example in a medical intervention, such as a minimally-invasive intervention). The adapter device is designed such that the position sensor(s) is/are or can be at a variable position with respect to the one or more ultrasound probes for one use event to another use event. The positioning or arrangement of the position sensor of the adapter device with respect to the ultrasound probe does not need to be repeatable. The position sensor(s) can be integrated into the adapter device or attached to the adapted device (e.g. glued to the adapter device). Alternatively, the position sensor(s) can be removably attached to or integrated into the adapter device (e.g. using a separate removable part having the position sensor(s)).

In one example, one adapter device is attachable or attached to exactly one of the ultrasound probes from one use event to another use event. However, from the one use event to the other use event the position sensor(s) is/are at a variable position with respect to that one single ultrasound probe, due to the imprecise manufacturing of the adapter device, e.g. due to tolerances.

In another example, the adapter device is attachable or attached to a first ultrasound probe for a first use event and a second, different ultrasound probe for a second use event. Due to the imprecise manufacturing of the adapter device (e.g. tolerances), the position sensor(s) is/are at a variable position with respect to the second ultrasound probe, compared to the first ultrasound probe from the first use event to the second use event. In other words, for the second use event, the position sensor(s) is/are at another position compared to the position of the position sensor(s) for the first use event.

Using the imprecisely manufactured adapter device in the (uncalibrated) system nevertheless works, as a special automatic self-calibration is used according to the invention. This automatic self-calibration automatically self-calibrates, for each use event, the imaging data with respect to the position sensor(s) by calculating a calibration matrix using an image based volume motion and a tracking based volume motion. The image based volume motion represents an image motion of at least two image volumes within the coordinate system and is derived from the imaging data. The tracking based volume motion represents a tracking motion of the image volumes within the coordinate system and is derived from the tracking data. With automatic self-calibration it is meant that no special pre-calibration (e.g. using a phantom) needs to be performed anymore. The tracking data and imaging data that is anyway generated during the use of the system, such as during a treatment or surgery, can be used for this calibration. In particular, the self-calibration can be performed during the intervention (e.g. surgery) itself. The self-calibration happens with no manual input from a user (e.g. doctor). The calibration happens with no changes to existing clinical workflow. The use of the imprecise adapter in combination with the automatic self-calibration method thus simplifies the clinical workflow.

Preferred embodiments of the invention are defined in the dependent claims. It shall be understood that the claimed volume-motion-based calibration method or a computer program has similar and/or identical preferred embodiments as the claimed ultrasound-image-guided system and as defined in the dependent claims.

In one embodiment the system is uncalibrated before the computation device automatically self-calibrates the imaging data with respect to the coordinate system of the at least one position sensor. Thus, the system can be uncalibrated before the use event. In this case the calibration matrix that is calculated is an initial calibration matrix. This means, that no calibration matrix for that specific ultrasound probe has been calculated before.

In another embodiment the adapter device is reusable for a plurality of use events. This reduces the costs of the system.

In a further embodiment wherein the adapter device is a hard shell having the least one position sensor integrated therein or attached thereto. This provides a robust adapter device.

In a variant of this embodiment the hard shell is separated into at least two parts adapted to be clamped against each other. This provides for a removable adapter device, which is in particular reusable for multiple use events.

In an alternative embodiment the adapter device is an elastic tube. This provides for an adapter device that optimally fits to the ultrasound probe.

In a variant of this embodiment the elastic tube is heat shrunk over the ultrasound probe. This provides for an easy and reliable way of attaching the adapter device to the ultrasound probe.

In another alternative embodiment the adapter device is an inelastic pre-form tube. This provides a robust adapter device.

In a variant of this embodiment the pre-form tube has an internal adhesive layer. This provides for an easy and reliable way of attaching the adapter device to the ultrasound probe.

In a further embodiment, each image volume is a distinct subset of a baseline image volume of the anatomical object. For example, the baseline image volume can be a full ultrasound volume scan of a heart.

In a further embodiment the image based volume motion is computed as a function of an image location of a first image volume within the coordinate system relative to an image location of a second image volume within the coordinate system. Alternatively or cumulatively, the tracking based volume motion is computed as a function of a tracked location of a first image volume within the coordinate system as represented by the tracking data and a tracked location of a second image volume within the coordinate system as represented by the tracking data.

In a variant of this embodiment, a computation of the image-based volume motion includes a registration between the first image volume and the second image volume, in particular to a baseline image volume of the anatomical object. Alternatively or cumulatively, the computation of the tracking based volume motion includes a registration transformation between the first volume image and the second volume image as a function of the tracked location of the first image volume within the coordinate system, the tracked location of the second image volume within the coordinate system and the calibration matrix.

In a further variant, the computation of the image-based volume motion includes a compensation for movement of the anatomical object within the coordinate system.

In another embodiment the tracking data and the imaging data are generated simultaneously. In particular, a number of image volumes of the anatomical object can be and a number of readings of a tracking signal via the at least one position sensor can be generated simultaneously, wherein each reading of the tracking signal corresponds to a generated image volume. The number can correspond to a number of different poses of the ultrasound probe. In this way, a number of motion pairs are provided, which can then be used for the calibration matrix calculation.

In another embodiment the computation device is operable to calculate the calibration matrix by solving a linear equation using the tracking based volume motion and the image based volume motion. In particular, the tracking based volume motion and the image based volume motion can be equated using the linear equation, since the amount of motion should be the same. Using such linear equation provides for a closed-form solution and a fast calibration. The computation cannot get trapped in local minima, as compared with nonlinear optimization methods for example. In a variant of this embodiment, the linear equation is solved using dual quaternion.

In a further embodiment the calibration matrix represents a spatial relationship between the image volumes and the at least one position sensor.

In another embodiment the at least one position sensor is an electromagnetic sensor and the tracking device is an electromagnetic tracking device. In an alternative embodiment, the at least one position sensor is an optical sensor and the tracking device is an optical tracking device. Any other suitable type of position sensor and tracking system can also be used, such as for example a FOSSL sensor and tracking system or a RFID sensor and tracking system.

In a further embodiment the computation device further operable to execute a validation testing of the calibration matrix derived from the automatic self-calibration, including a testing of an absolute differential between the image based volume motion and the tracking based volume motion. This provides for an intra-operative quality control of the ultrasound probe, more particular the calibration, during a medical intervention, such as a surgical procedure (e.g. a cardiac procedure). In particular, the validity of the calibration matrix can be continuously tested. If at any point, the calibration matrix becomes invalid for any reason, a warning sign may be raised by the system.

### BRIEF DESCRIPTION OF THE DRAWINGS

These and other aspects of the invention will be apparent from and elucidated with reference to the embodiment(s) described hereinafter. In the following drawings
Fig. 1 illustrates an exemplary embodiment of an ultrasound-image-guided system in accordance with the present invention;
Fig. 2 illustrates an exemplary volume motion of two (2) image volumes of an anatomical object as known in the art;
Fig. 3a illustrates a first embodiment of an adapter device of a system in accordance with the present invention;
Fig. 3b illustrates a second embodiment of an adapter device of a system in accordance with the present invention;
Fig. 4 illustrates an exemplary operation of the ultrasound-image-guided system in accordance with the present invention;
Fig. 5 illustrates a flowchart representative of a volume-motion-based calibration method in accordance with a first embodiment of the present invention;
Fig. 6 illustrates a flowchart representative of an exemplary embodiment of an image based volume motion computation method in accordance with the present invention;
Figs. 7A and 7B illustrate flowcharts representative of two (2) exemplary embodiments of an image based registration method in accordance with the present invention;
Fig. 8 illustrates a flowchart representative of a first exemplary embodiment of a heart motion modeling method in accordance with the present invention;
Fig. 9 illustrates a flowchart representative of an exemplary embodiment of a tracking based volume motion computation method in accordance with the present invention;
Fig. 10 illustrates an exemplary operation of the ultrasound-image-guided system in accordance with a second embodiment;
Fig. 11 illustrates a flowchart representative of a volume-motion-based calibration method in accordance with a second embodiment;
Fig. 12 illustrates a flowchart representative of an exemplary embodiment of a calibration threshold computation method;
Fig. 13 illustrates an exemplary operation of the ultrasound-image-guided system or the volume-motion-based calibration method in accordance with the present invention in a clinical context; and
Fig. 14a and Fig. 14b each illustrate results obtained with the system or method in accordance with the present invention.

### DETAILED DESCRIPTION OF THE INVENTION

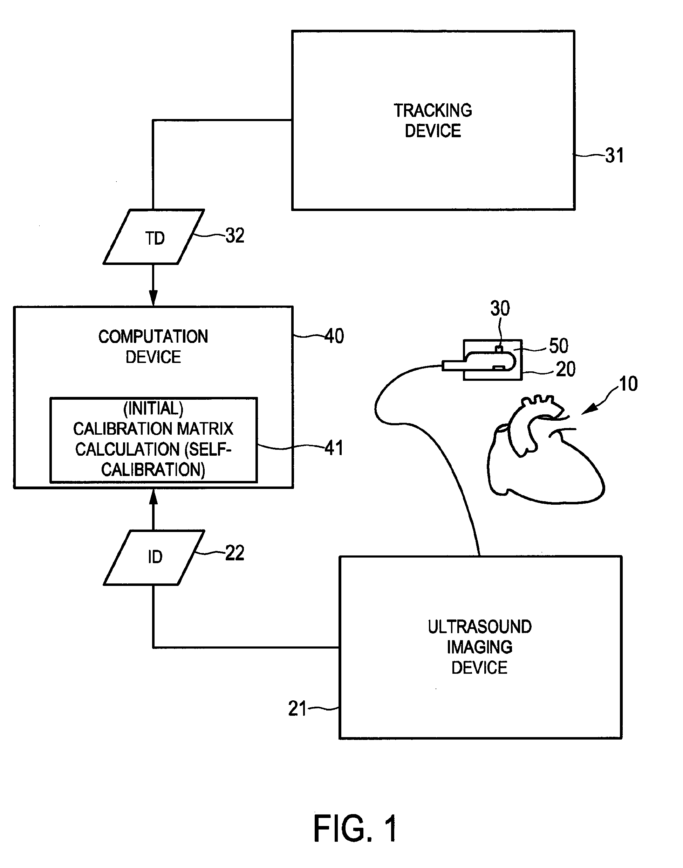

Fig. 1 illustrates an exemplary embodiment of an ultrasound-image-guided system. The system employs an ultrasound imaging system, a tracking system and a computation device 40.

For purposes of the invention, the ultrasound imaging system is broadly defined herein as any system including one or more ultrasound probes 20 operable or structurally configured to generate image volumes of an anatomical object (e.g., a heart 10) within a coordinate system, and an ultrasound imaging device 21 operable or structurally configured to generate imaging data 22 of the anatomical object based on the image volumes (processing the image volumes). In particular, each image volume can be a district subset of a baseline image volume of the anatomical object. The ultrasound imaging system can particularly use a 3D trans-esophageal echo ("TEE") probe. In one embodiment, the iEEE intelligent echo system commercially sold by Philips Healthcare may serve as an ultrasound imaging system. However, any other suitable ultrasound imaging system can be used.

For purposes of the present invention, the tracking system is broadly defined herein as any system including an adapter device 50 comprising at least one position sensor 30, and a tracking device operable or structurally configured to generate tracking data 32 representative of a tracking of the at least one position sensor 30 within a coordinate system (track position sensor(s) 30 within the coordinate system). The adapter device 50 is, for one use event, attachable or attached to one of the ultrasound probes 20. A use event refers to the attachment of the adapter device 30 to one of the ultrasound probes and the use of this adapter-probe combination. The adapter device 30 is designed such that the at least one position sensor 30 is at a variable position with respect to the one or more ultrasound probes from one use event to another use event. Thus, the adapter device can be imprecisely manufactured. Examples of the tracking system include, but are not limited to, any type of electromagnetic tracking system and any type of optical tracking system, for example shape sensing. In one embodiment, the Aurora™ Electromagnetic Tracking System commercially sold by NDI may serve as an electromagnetic tracking system. However, any other suitable tracking system can be used.

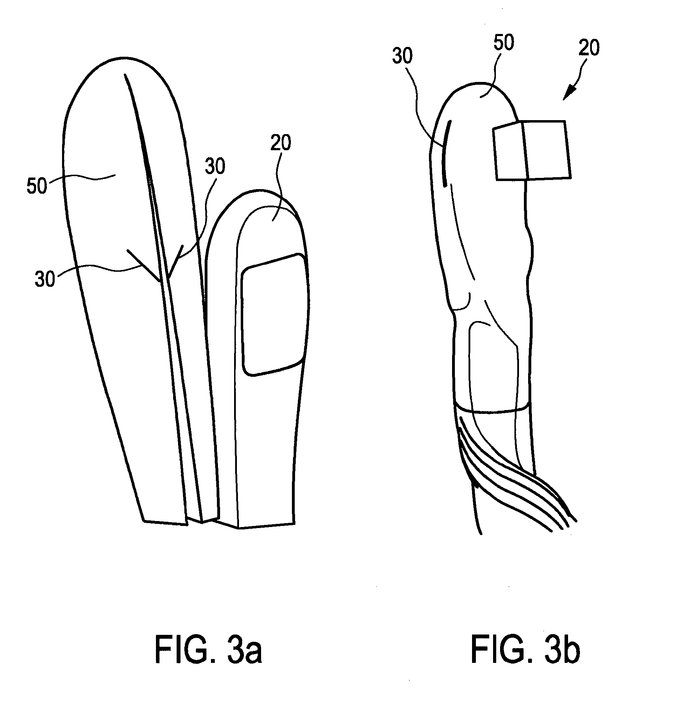

Fig. 3a illustrates a first embodiment of an adapter device and Fig. 3b illustrates a second example of an adapter device. In the embodiment of Fig. 3a, the adapter device 50 is a hard shell having two position sensors 30 integrated therein or attached thereto. In the embodiment of Fig. 3a and Fig. 3b, the position sensors 30 are electromagnetic (EM) sensors. The hard shell shown in Fig. 3a is separated into two parts adapted to be clamped against each other. The two parts can be hold apart, placed over the ultrasound probe 20 and then be clamped against each other. In this way, the adapter device fully encloses the ultrasound probe. The adapter device 50 and ultrasound probe 20 are adapted for in-vivo application or use. In the embodiment of Fig. 3b the adapter device 50 is an elastic tube. The elastic tube is heat shrunk over the sound probe 20.

For purposes of the present invention, computation device 40 is broadly defined herein as any device operable or structurally configured to automatically self-calibrate, for each use event, the imaging data 22 with respect to the coordinate system of the at least one position sensor 30 by calculating a calibration matrix using an image based volume motion and a tracking based volume motion. This can be performed in a calibration unit 41 of the computation device 40, as illustrated in Fig. 1. The computation device 40 can further be operable to register the image volumes to the baseline image volume of the anatomical object 10 (e.g., a full US volume of heart 10). To this end, a calibration matrix is utilized by computation device 40 as a transformation that converts the coordinates of the voxels in the image volumes in the coordinate system for tracking position sensor 30. In other words, a calibration matrix represents a spatial relationship between the image volumes and the at least one position sensor 30.

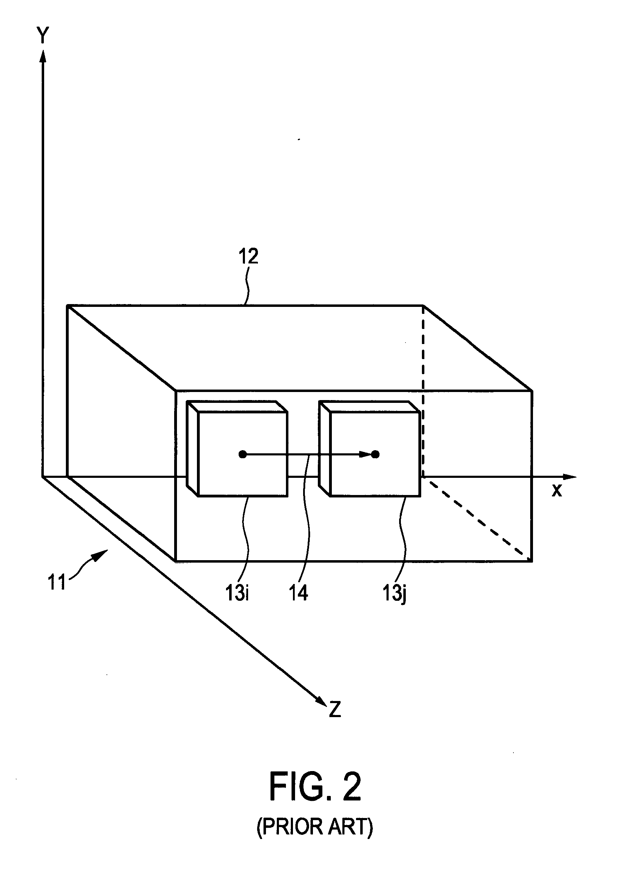

To facilitate an understanding of the calibration matrix, Fig. 2 illustrates a baseline image volume 12 of an anatomical object (e.g., a full US volume scan of a heart) within a coordinate system 11 (e.g., a tracking coordinate system). Ultrasound probe 20 (Fig. 1) is operated to sequentially generate a volume image 13i and a volume image 13j, and position sensor 30 (Fig. 1) is tracked within coordinate system 11 as volume images 13 are generated by probe 20. In practice, volume images 13 may overlap, but are segregated in Fig. 2 for purposes of clearly showing each individual volume image 13.

The calibration matrix provides a transformation that converts the coordinates of the voxels in image volumes 13 into coordinate system 11. This enables image volumes 13 to be mapped into the coordinate system for image reconstruction purposes. For the automatic self-calibration, the computation device 40 measures motion 14 between image volumes 13 from two sources. The first source being an image motion of image volumes 13, and the second source being a tracking motion of image volumes 13. Thus, the image volume motion is measured from two sources, (a) image based volume motion and (b) tracking based volume motion. The image based volume motion, thus, represents an image motion of at least two volumes derived from the imaging system, and the tracking based volume motion represents a tracking motion of the image volumes.

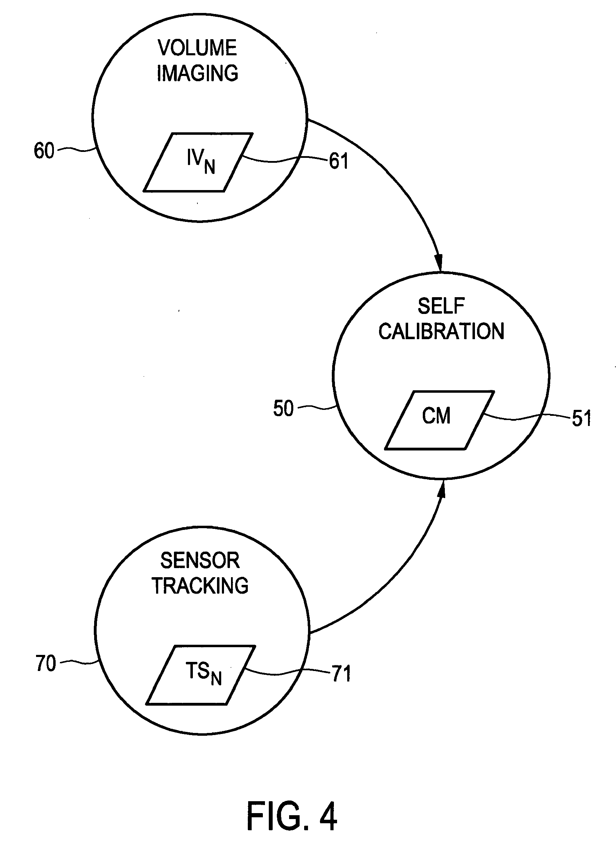

A description of Figs. 4-9 will now be provided herein to provide a more detailed explanation of the automatic self-calibration in accordance with the present invention.

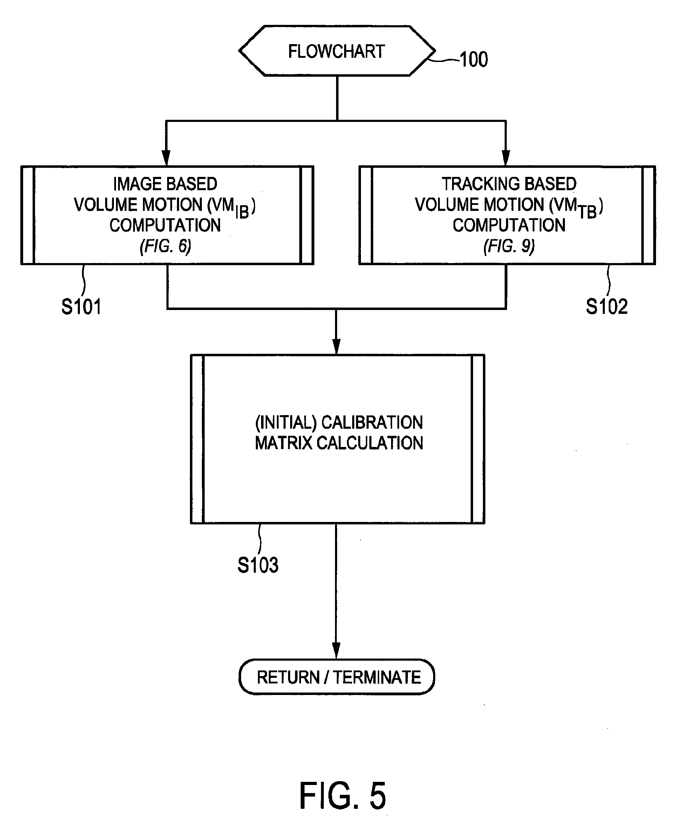

Fig. 4 illustrates various exemplary operation states of the ultrasound-image-guided system. Initially, there is a volume imaging state 60 for generating a number N of image volumes 61 of the anatomical object (e.g., heart 10) via probe 20 (Fig. 1), and a sensor tracking state 70 for a number N of readings of a tracking signal 71 via position sensor 30 (Fig. 1) with each reading of tracking signal 71 corresponding to a generated image volume 61. This corresponds to the generation of imaging data 22 and to the generation of tracking data 32. This data is then used in s self-calibration state 50, as shown in Fig. 4. In self-calibration state 50 an automatic self calibration, for that use event, of the imaging data 22 with respect to the coordinate system 11 is performed by calculating a calibration matrix 51 using an image based volume motion and a tracking based volume motion. The calibration matrix 51 can in particular be an initial calibration matrix. The accuracy of calibration matrix 51 is essential for locating each image volume within the coordinate system via tracking signal 71. State 50 is implemented by a volume-motion-based calibration method executed by computation device 40, as further explained herein in connection with the description of Figs. 5-9.

Fig. 5 illustrates a flowchart 100 representative of one embodiment of the volume-motion-based calibration method. A stage S101 of flowchart 100 encompasses a computation by the computation device 40 of an image based volume motion VM_{IB}, and a stage S 102 of flowchart 100 encompasses a computation by the computation device 40 of a tracking based volume motion VM_{TB}. For purposes of the present invention, image based volume motion VM_{IB} is broadly defined herein as any motion between image volumes 61 (Fig. 4) of the anatomical object within a coordinate system (e.g., coordinate system 11 shown in Fig. 2) derived from imaging data 22 (Fig. 1) of image volumes 61, and tracking based volume motion VM_{TB} is broadly defined herein as any motion between image volumes 61 of the anatomical object within the coordinate system derived from tracking data 32 (Fig. 1). Stage S103 of flowchart 100 encompasses an initial calibration matrix calculation using the image based volume motion VM_{IB} and to tracking based volume motion VM_{TB}. In particular, the calibration matrix can be calculated by formulating a motion-based calibration problem as a linear equation AX=BX, where X is the calibration matrix (e.g. calibration transformation from ultrasound image space US to sensor space S, represented by *X = T* _{S•} *us),* A stands for the tracking based volume motion VM_{TB} (e.g. motion from a pose 2 to a pose 1, represented by A = (*T_{S• S1}*)*⁻¹ T_{S• S2}*), and B stands for the image based volume motion (e.g. motion from a ultrasound image US2 to a position ultrasound image US1, represented by B = *T _{US1• US2},* where USᵢ corresponds to a pose i). In particular, the tracking based volume motion VM_{TB} and the image based volume motion VM_{IB} can be equated using the linear equation, since the amount of motion should be the same. Using such a linear equation provides for a closed-form solution and a fast calibration. The computation cannot get trapped in local minima compared with nonlinear optimization methods for example. In a variant of this embodiment, the linear equation is solved using a dual quaternion.

In one example the linear equation can be solved using dual quaternion. Such dual quaternion is for example described in Daniilisdis K, 1999, "Hand-eye calibration using dual quaternion", The Int. J. of Robotics Research, 18(3):286-298.

An exemplary computation algorithm can for example comprise to provide motion pairs Aᵢ, Bᵢ, providing a screw representation of the motion (using motion pairs Aᵢ, Bᵢ ) which yields a matrix T = [S1, ..., Sn], performing a singular value decomposition SVD of the matrix T, and providing the calibration matrix X as a function of the singular value decomposition, X = f(SVD).

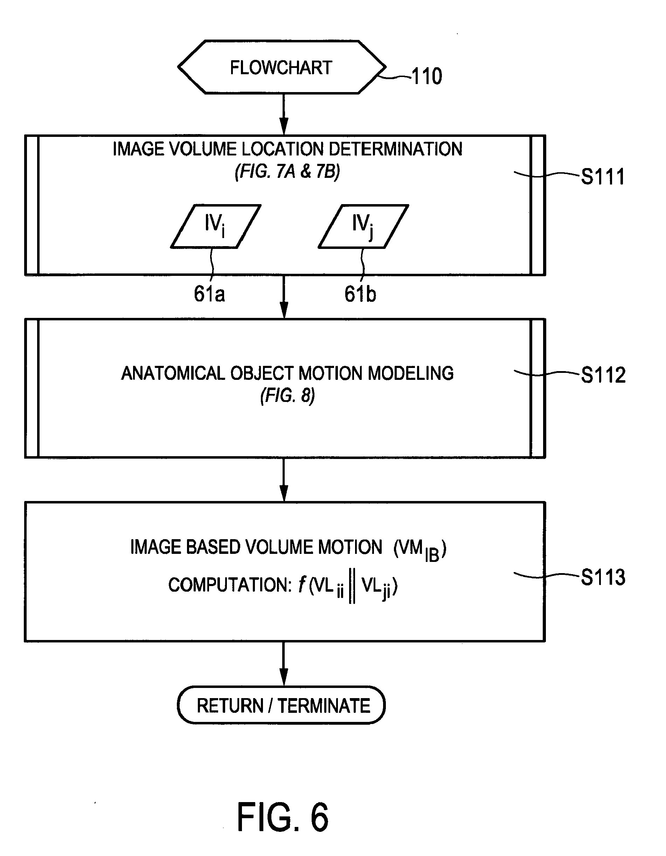

Fig. 6 illustrates a flowchart 110 representative of an image based volume motion computation method that may be executed during stage S101 (Fig. 5). This method involves a processing of pair (i, j) of image volumes (e.g., image volumes 13 shown in Fig. 2). Specifically, a stage S111 of flowchart 110 encompasses a determination of a location of an image volume 61a and an image volume 61b within the coordinate system (e.g., coordinate system 11 shown in Fig. 2), and a stage S112 of flowchart 110 encompasses a motion compensation of the determined locations of image volumes 61a and 61b in view of a modeling of a motion of the anatomical object (e.g., heart 10).

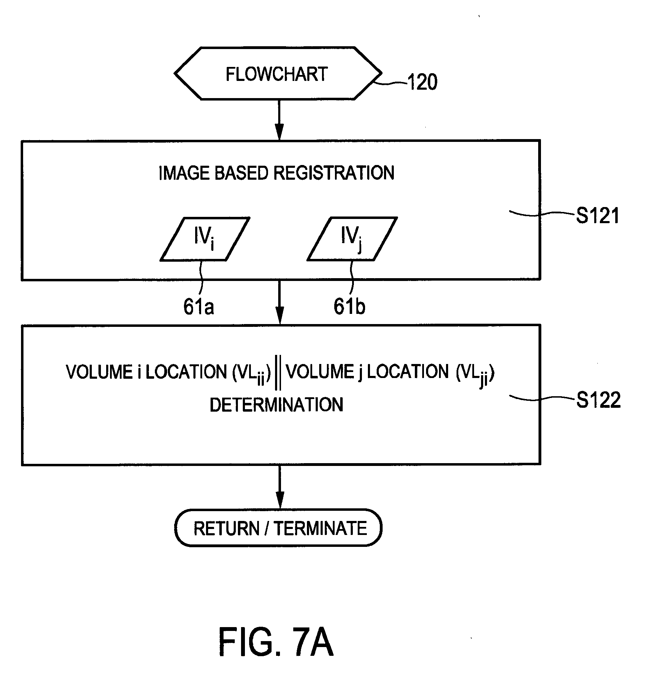

In one embodiment of stage S111 (Fig. 6), a flowchart 120 as shown in Fig. 7A includes a stage S121 encompassing an image based registration of the pair (i, j) of image volumes 61a and 61b via a known image based rigid or deformable registration and known optimization metrics (e.g., mutual information, cross correlation, etc.). Flowchart 120 further includes a stage S122 encompassing a utilization of the registration of image volumes 61a and 61b to determine a location VLᵢᵢ of image volume 61a within the coordinate system relative to a location VLⱼᵢ of image volume 61b within the coordinate system.

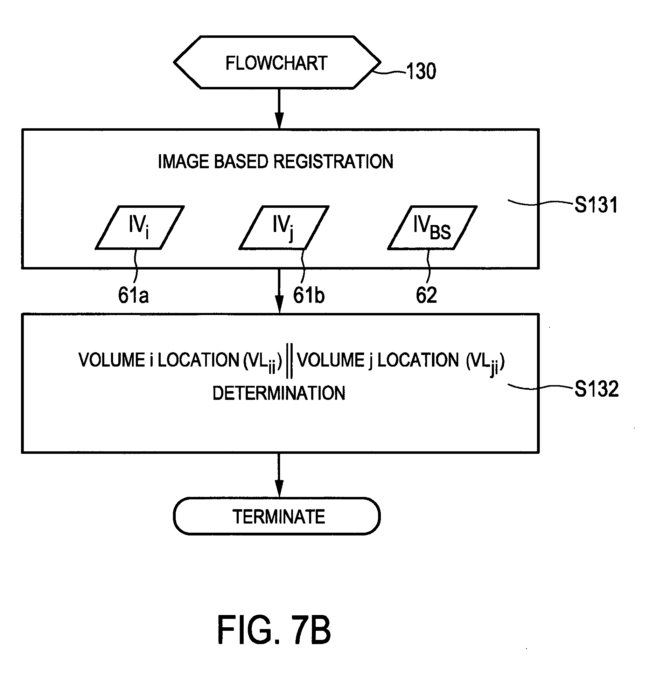

In an alternative embodiment of stage S111 (Fig. 6), a flowchart 130 as shown in Fig. 7B includes a stage S131 encompassing an image based registration of the pair (i, j) of image volumes 61a and 61b to a baseline image volume 62 of the anatomical object (e.g., a full US image). These registrations may be performed via an image based rigid or deformable registration and known optimization metrics (e.g., mutual information, cross correlation, etc.) Flowchart 130 further includes a stage S132 encompassing a utilization of the registration of image volume 61a to baseline image volume 62 to determine location VLᵢᵢ of image volume 61a relative to baseline image volume 62 within the coordinate system. Similarly, the registration of image volume 61b to baseline image volume 62 is utilized to determine a location VLⱼᵢ of image volume 61b relative to the baseline image volume 62 within the coordinate system. This facilitates a determination of location VLᵢᵢ of image volume 61a relative to location VLⱼᵢ of image volume 61b within the coordinate system.

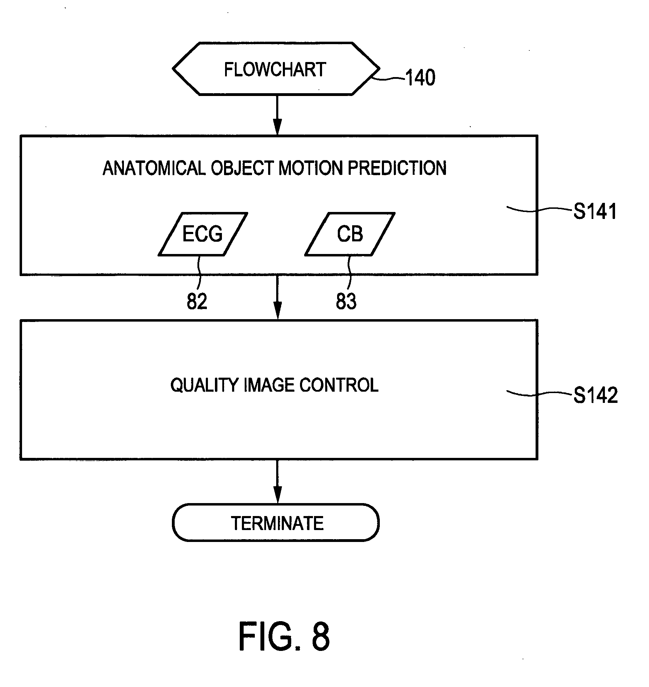

In one embodiment of stage S112 (Fig. 6), a flowchart 140 as shown in Fig. 8 includes a stage S141 encompassing a prediction of the motion of anatomical object within the coordinate system. For example, with the anatomical object being heart 10, a known learning algorithm utilizing an electrocardiogram signal 82 for cardiac phase, a chest belt signal 83 for respiratory phase and any other additional sensing signals to predict the motion of heart 10 within the coordinate system can be used. Flowchart 140 further includes a stage S142 encompassing a quality image control involving a motion compensation of image volumes 61a and 61b via the predicted motion of the anatomical object. In one embodiment with the anatomical object being heart 10, image volumes 61 corresponding to a diastolic phase of heart 10 via ECG signal 82 are exclusively utilized by stage S113 (Fig. 6) for quality control purposes and stage S103 (Fig. 5) will only process the volume motions of these selected image volumes 61. Please note this selection assume respiratory motion is minimal.

In an alternative embodiment, image volumes 61 at time intervals when respiratory phase and cardiac phase come back to the same cycle are exclusively utilized by stage S113 (Fig. 6) for quality control purposes and stage S103 (Fig. 5) will only process the volume motions of these selected image volumes 61.

Referring back to Fig. 6, a stage S113 of flowchart 110 encompasses a computation of an image based volume motion VM_{IB} as a function of the location VLᵢᵢ of image volume 61a within the coordinate system relative to the location VLⱼᵢ of image volume 61b within the coordinate system as known in the art. The computed image based volume motion VM_{IB} is implemented by stage S103 (Fig. 5) during the initial calibration matrix calculation.

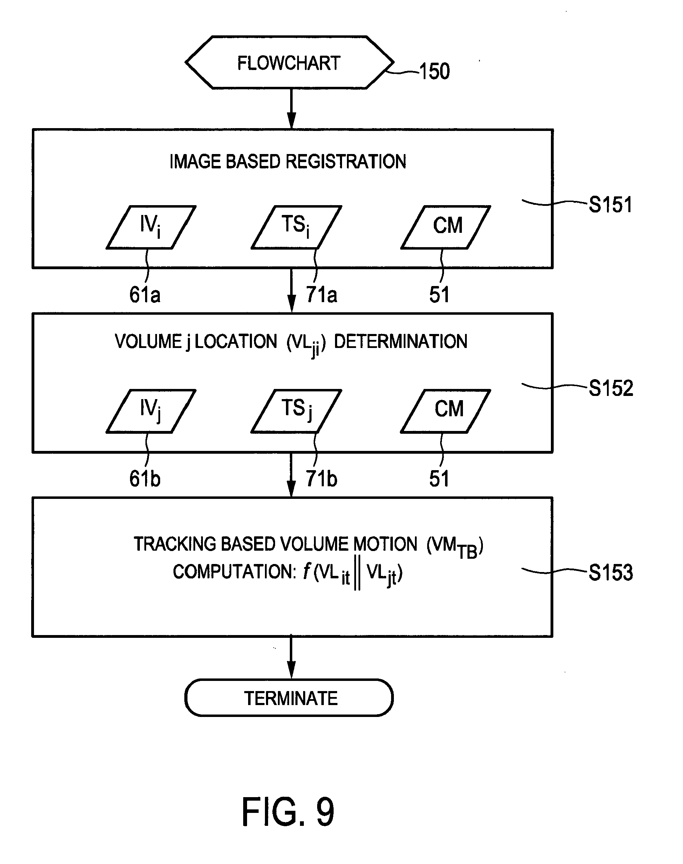

Fig. 9 illustrates a flowchart 150 representative of a tracking based volume motion computation method that may be executed during stage S102 (Fig. 5). A stage S151 of flowchart 150 encompasses a determination of a location VLᵢₜ of image volume 61a within the coordinate system via a tracking signal 71a and calibration matrix 51 as known in the art. The determined location of VLᵢₜ of image volume 61a may be confirmed with a location of the baseline image volume of the anatomical object.

A stage S152 of flowchart 150 encompasses a determination of a location VLⱼₜ of image volume 61b within the coordinate system via a tracking signal 71b and calibration matrix 51 as known in the art. The determined location of VLⱼₜ of image volume 61b may be confirmed with a location of the baseline image volume of the anatomical object.

A stage S153 of flowchart 150 encompasses a computation of the tracking based volume motion VM_{TB} as a function of location VLᵢₜ of image volume 61a within the coordinate system relative to a location VLⱼₜ of volume 61b within the coordinate system as known in the art. In one embodiment, a registration transformation between image volumes 61a and 61b based on location VLᵢₜ of image volume 61a, location VLⱼₜ of volume 61b and calibration matrix 51 may be executed as known in the art during stage S153. This computed tracking based volume motion VM_{TB} is implemented by stage S103 (Fig. 5) during the initial calibration matrix calculation.

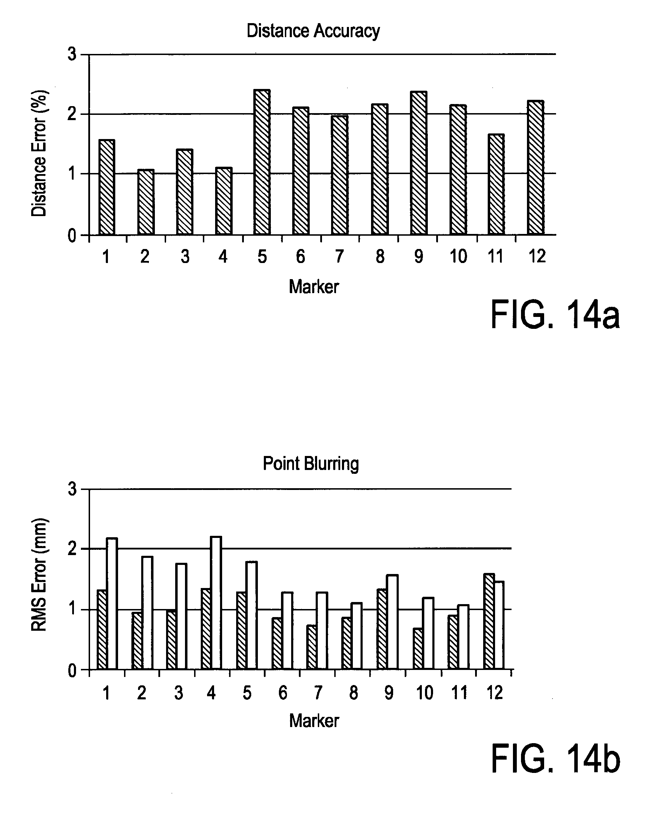

Fig. 14a and 14b each illustrate results obtained with the ultrasound-image-guided system and/or the volume-motion-based calibration method described above. The results of the performance of the calibration were obtained by visual inspection and quantitative validation using a heart simulating object with fiducial markers, simply to test the performance of the system /bmethod. These markers were localized in both a computer tomography image and an ultrasound image and then calibration validation metrics were calculated, including point blowing of single markers over multiple measurements, as shown in Fig. 14b, and distance accuracy of multiple markers, as shown in Fig. 14a. The positions of these markers in the computer tomography images were used for the gold standard due to high quality computer tomography images of the hard-simulating object. As can be seen from Fig. 14a and Fig. 14b, the result was that the performance of calibration was very accurate.

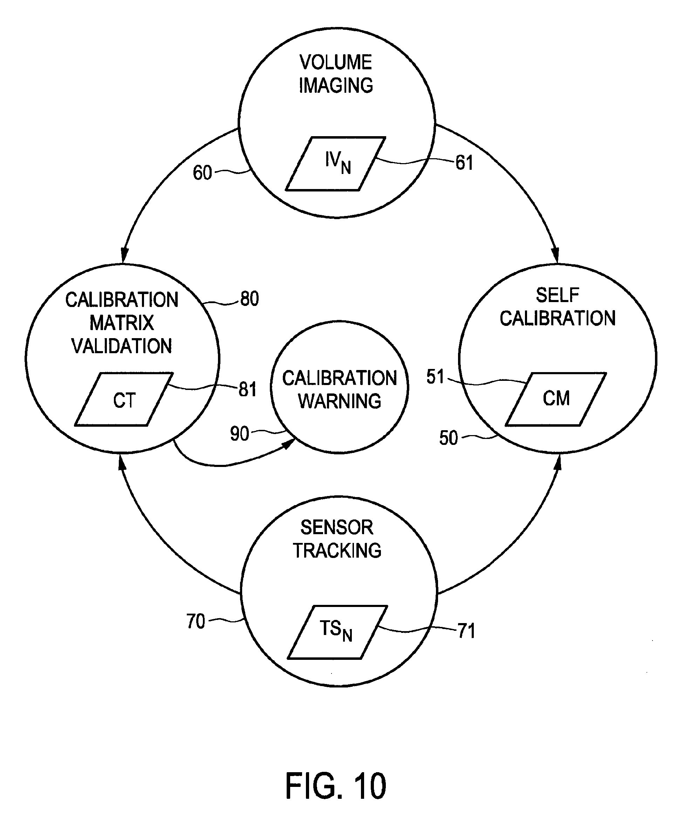

Figs. 10-12 illustrate another (second) embodiment of the system and method in accordance with the present invention. The embodiment basically corresponds to the first embodiment described herein above, but in combination with a validity testing of the calibration matrix. The calibration matrix calculated by the automatic self-calibration may become inaccurate for a variety of reasons, such as, for example, unexpected field distortions, accidental physical movement of position sensor 30 relative to probe 20 and a partial breakdown of position sensor 30. To test the validity of the calibration matrix, the computation device 40 again measures motion 14 between image volumes 13 from two sources, an image based volume motion and a tracking based volume motion.

A description of Figs. 10-12 will now be provided herein to provide a more detailed explanation of the validity testing of the calibration matrix.

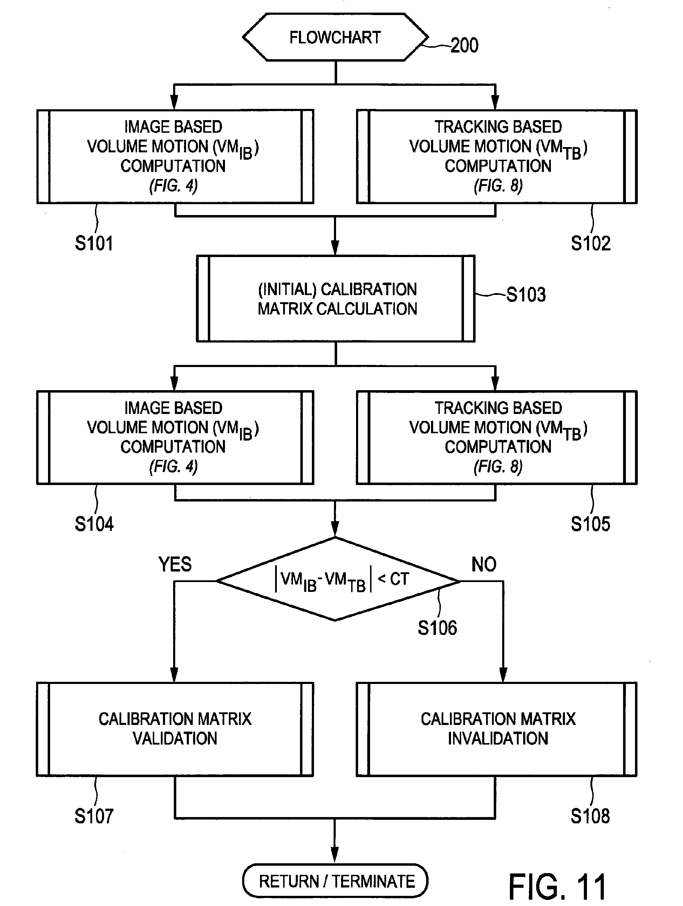

Fig. 10 illustrates the operational states of the ultrasound image-guided system as explained with reference to Fig. 4. Further, the system moves from self-calibration state 50 to a calibration matrix validation state 80. The accuracy of calibration matrix 51 is essential for locating each image volume 61 within the coordinate system via tracking signal 71. Thus, the calibration validation state 80 utilizes image volumes 61 and tracking signal 71 to ascertain the validity of the calibration matrix. State 80 proceeds to a calibration warning state 90 in view of an invalid calibration matrix. State 80 can be implemented by a calibration matrix validation testing method executed by the computation device 40, as further explained herein in connection with the description of Figs. 11-12.

Fig. 11 illustrates a flowchart 200 representative of the second embodiment of the calibration method in accordance with the present invention, in combination with a calibration matrix validation testing method. Steps S101, S102 and S103 correspond to the steps as explained with reference to Fig. 4. Additionally, a stage S104 of flowchart 200 encompasses another computation by computation device 40 of an image based volume motion VM_{IB}, and a stage S105 of flowchart 100 encompasses another computation by computation device 40 of a tracking based volume motion VM_{TB}.

Stage S106 of flowchart 100 encompasses a testing of an absolute differential between image based volume motion VM_{IB} and tracking based volume motion VM_{TB} relative to a calibration threshold CT. If the absolute differential is less than calibration threshold CT, then a stage S107 of flowchart 200 encompasses a validation of the calibration matrix that facilitates the continual generation of image volumes 61. Conversely, if the absolute differential is not less than calibration threshold CT, then a stage S108 of flowchart 200 encompasses an invalidation of the calibration matrix that facilitates a warning as to the probable distortion or inaccuracy of image volumes 61.

In one exemplary embodiment of stages S107 and S108, real-time calibration alarm is deactivated as the image volumes 61 are being generated with a valid calibration matrix and is activated as a warning to the probable distortion or inaccuracy of image volumes 61 upon an invalidation of the calibration matrix. In an exemplary embodiment of stage S108, a regional map of the anatomical object is displayed as a warning to the probable distortion or inaccuracy of image volumes 61 associated with the regional map. In another exemplary embodiment of stages S107 and S108, a map of the anatomical object may be displayed, whereby region(s) of the map associated with an invalid calibration matrix is (are) distinguished from region(s) of the map associated with a valid calibration matrix as a means for providing a warning of probable distortion or inaccuracy of image volumes 61 associated with the invalid region(s).

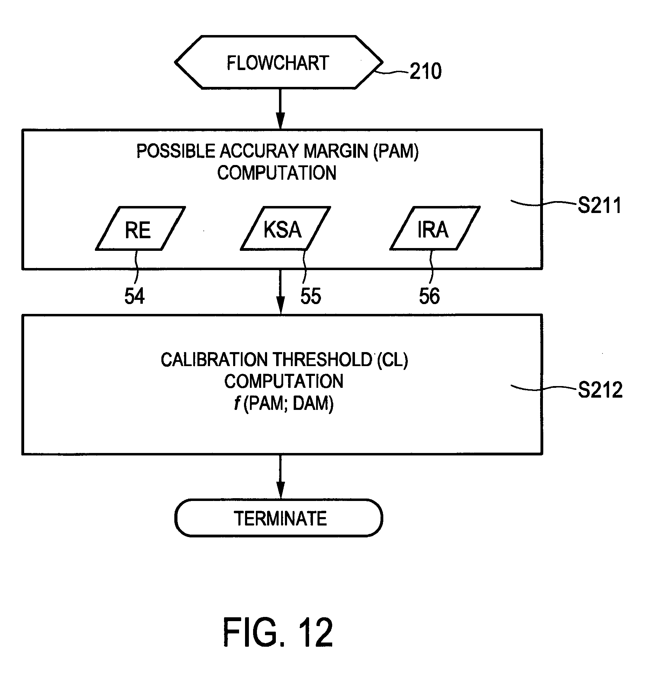

Fig. 12 illustrates a flowchart 210 representative of a calibration threshold computation method. A stage 211 of flowchart 210 encompasses a computation of a possible accuracy margin of the calibration matrix. Random error information 54 can be associated with the tracking system, known statistical accuracy data 55 associated with a pre-operative calibration process, and an image registration accuracy data 56 may be utilized in computing the possible accuracy margin. A stage 212 of flowchart 200 encompasses a computation of calibration threshold CL as a function of the computed possible accuracy margin and a desired accuracy margin associated with the application of the system.

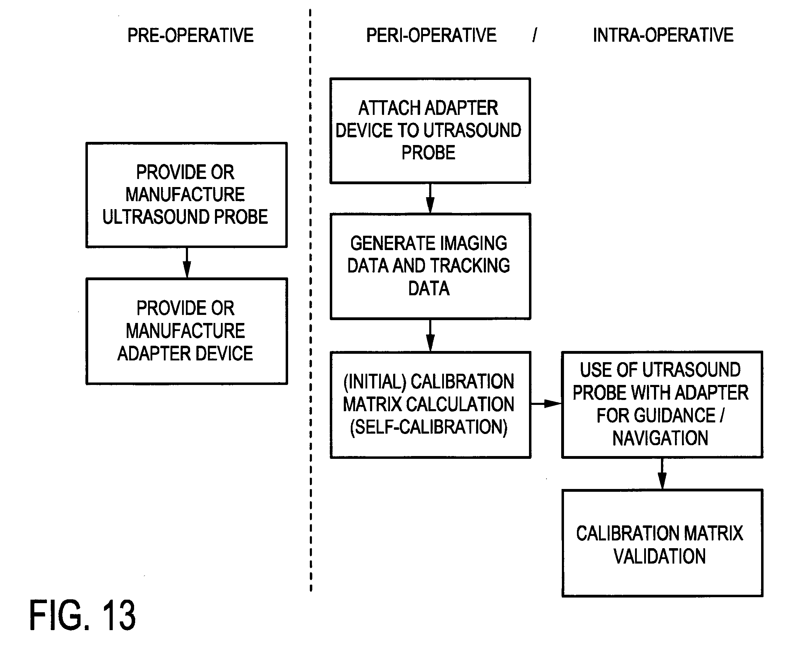

Fig. 13 illustrates an exemplary operation of the ultrasound-image-guided system or the volume-motion-based calibration method in accordance with the present invention in a clinical context. Being operatively, the ultrasound probe 20 is provided or manufactured, and the adapter device 50 is provided or manufactured (in particular, the ultrasound probe and adapter device as previously described). Then, the surgery is started. In the first minutes of the surgery (peri-operatively) the adapter device 50 is attached to the ultrasound probe 20. Thus, the (imprecisely manufactured) adapter device 50 disclosed herein can be (inaccurately) attached to the ultrasound probe 20 just before the surgery. This can for example be the hard shell that is clamped on the ultrasound probe and described in connection with Fig. 3a. Then, normal use of the adapter device/ultrasound probe combination is performed on the patient, thus generating image data and tracking data. This data is then used intra-operatively to automatically self-calibrate the system or ultrasound probe with respect to the position sensor. This calibrated ultrasound probe with adapter (adapter device-ultrasound probe) is then used for navigation and guidance of a surgical instrument, such as a needle or a catheter for example, during the surgery. Intra-operatively, the calibration matrix can be validated for quality monitoring. In particular, the adapter device and ultrasound probe are adapted for in-vivo application or use in this case. For surgery, the surgical instrument can be placed inside the ultrasound image/volume. Alternatively, the surgical instrument outside of the ultrasound image/volume, and can in particular be used for planning and targeting.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. An ultrasound-image-guided system, the system comprising:
- one or more ultrasound probes (20) operable to generate image volumes (13i, 13j) of an anatomical object (10);
- an adapter device (50) comprising at least one position sensor (30);
- a tracking device (51) operable to generate tracking data (32) representative of a tracking of the at least one position sensor (30) within a coordinate system (11);
- an ultrasound imaging device (21) operable to generate imaging data (22) of the anatomical object (10) based on the image volumes (13i, 13j); and
- a computation device (40) operable to automatically self-calibrate, for each use event, the imaging data (22) with respect to the coordinate system (11) of the at least one position sensor (30) by calculating a calibration matrix (51) using an image based volume motion (VM_{IB}) and a tracking based volume motion (VM_{TB}),
the image based volume motion (VM_{IB}) representing an image motion of at least two image volumes (13i, 13j) derived from the imaging data (22),
the tracking based volume motion (VM_{TB}) representing a tracking motion of the image volumes (13i, 13j) derived from the tracking data (32)
charachterized in that
the adapter device (50) is for one use event, attachable to one of the ultrasound probes (20), and in that
the computation device (40) further operable to execute a validation testing of the calibration matrix (51) derived from the automatic self-calibration, including a testing of an absolute differential between the image based volume motion (VM_{IB}) and the tracking based volume motion (VM_{TB}).

2. The system of claim 1, wherein the system is uncalibrated before the computation device (40) automatically self-calibrates the imaging data (22) with respect to the coordinate system (11) of the at least one position sensor (30).

3. The system of claim 1, wherein the adapter device (50) is reusable for a plurality of use events.

4. The system of claim 1, wherein the adapter device (50) is a hard shell having the least one position sensor (30) integrated therein or attached thereto.

5. The system of claim 4, wherein the hard shell is separated into at least two parts adapted to be clamped against each other.

6. The system of claim 1, wherein the adapter device (50) is an elastic tube.

7. The system of claim 6, wherein the elastic tube is heat shrunk over the ultrasound probe.

8. The system of claim 1, wherein the adapter device (50) is an inelastic pre-form tube.

9. The system of claim 8, wherein the pre-form tube has an internal adhesive layer.

10. The system of claim 1, wherein the image based volume motion (VM_{IB}) is computed as a function of an image location (VLᵢᵢ) of a first image volume (13i) within the coordinate system (11) relative to an image location (VLⱼᵢ) of a second image volume (13j) within the coordinate system (11) and/or wherein the tracking based volume motion (VM_{TB}) is computed as a function of a tracked location (VLᵢₜ) of a first image volume (13i) within the coordinate system (11) as represented by the tracking data (32) and a tracked location (VLⱼₜ) of a second image volume (13j) within the coordinate system (11) as represented by the tracking data (32).

11. The system of claim 1, wherein the computation device (40) is operable to calculate the calibration matrix by solving a linear equation using the tracking based volume motion and the image based volume motion.

12. The system of claim 1, wherein the at least one position sensor (30) is an electromagnetic sensor and the tracking device (51) is an electromagnetic tracking device.

13. A volume-motion-based calibration method for operating an ultrasound-image-guided system, the system comprising one or more ultrasound probes (20) operable to generate image volumes (13i, 13j) of an anatomical object (10), and an adapter device (50) comprising at least one position sensor (30), wherein the at least one position sensor (30) is at a variable position with respect to the one or more ultrasound probes (20) from one use event to another use event, the method comprising the steps of:
a) generating tracking data (32) representative of a tracking of the at least one position sensor (30) within a coordinate system (11);
b) generating imaging data (22) of the anatomical object (10) based on the image volumes (13i, 13j); and
c) automatically self-calibrating, for each use event, the imaging data (22) with respect to the coordinate system (11) of the at least one position sensor (30) by calculating a calibration matrix (51) using an image based volume motion (VM_{IB}) and a tracking based volume motion (VM_{TB}),
the image based volume motion (VM_{IB}) representing an image motion of at least two image volumes (13i, 13j) within the coordinate system (11) derived from the imaging data (22),
the tracking based volume motion (VM_{TB}) representing a tracking motion of the image volumes (13i, 13j) within the coordinate system (11) derived from the tracking data (32),
charachterized in that the adapter device (50) is for one use event, attachable to one of the ultrasound probes (20), and in that the method further comprises a step of
testing of the calibration matrix (51) derived from the automatic self-calibration, including a testing of an absolute differential between the image based volume motion (VM_{IB}) and the tracking based volume motion (VM_{TB}),

14. Computer program comprising program code means for causing a computer to carry out the steps of the method as claimed in claim 13 when said computer program is carried out on the computer.

## Patentansprüche

1. Ultraschallbildgeführtes System, das Folgendes umfasst:
- einen oder mehrere Ultraschallköpfe (20), die so funktionieren, dass sie Bildvolumen (13i, 13j) eines anatomischen Objekts (10) erzeugen,
- einen Adapter (50) mit zumindest einem Lagesensor (30),
- eine Tracking-Vorrichtung (51), die so funktioniert, dass sie Trackingdaten (32) erzeugt, die das Tracking des zumindest einen Lagesensors (30) in einem Koordinatensystem (11) darstellen,
- eine Ultraschall-Bildgebungsvorrichtung (21), die so funktioniert, dass sie auf der Grundlage der Bildvolumen (13i, 13j) Bilddaten (22) des anatomischen Objekts (10) erzeugt, und
- eine Berechnungsvorrichtung (40), die so funktioniert, dass sie bei jedem Einsatzereignis eine automatische Selbstkalibrierung der Bilddaten (22) in Bezug auf das Koordinatensystem (11) des zumindest einen Lagesensors (30) durchführt, indem sie unter Verwendung einer bildbasierten Volumenbewegung (VM_{IB}) und einer trackingbasierten Volumenbewegung (VM_{TB}) eine Kalibrierungsmatrix (51) berechnet,
wobei die bildbasierte Volumenbewegung (VM_{IB}) eine Bildbewegung von zumindest zwei Bildvolumen (13i, 13j) darstellt, die von den Bilddaten (22) abgeleitet wird,
wobei die trackingbasierte Volumenbewegung (VM_{TB}) eine Trackingbewegung der Bildvolumen (13i, 13j) darstellt, die von den Trackingdaten (32) abgeleitet wird,
**dadurch gekennzeichnet, dass**
der Adapter (50) bei einem Einsatzereignis an einem der Ultraschallköpfe befestigt werden kann und dass
die Berechnungsvorrichtung (40) ferner so funktioniert, dass sie eine Gültigkeitsprüfung der aus der automatischen Selbstkalibrierung abgeleiteten Kalibrierungsmatrix (51) einschließlich einer Prüfung einer absoluten Differenz zwischen der bildbasierten Volumenbewegung (VM_{IB}) und der trackingbasierten Volumenbewegung (VM_{TB}) ausführt.

2. System nach Anspruch 1, wobei das System unkalibriert ist, bevor die Berechnungsvorrichtung (40) die automatische Selbstkalibrierung der Bilddaten (22) in Bezug auf das Koordinatensystem (11) des zumindest einen Lagesensors (30) durchführt.

3. System nach Anspruch 1, wobei der Adapter (50) für eine Vielzahl von Einsatzereignissen wiederverwendbar ist.

4. System nach Anspruch 1, wobei der Adapter (50) eine Hartschale mit zumindest einem in sie integrierten oder an ihr befestigten Lagesensor (30) ist.

5. System nach Anspruch 4, wobei die Hartschale in zumindest zwei Teile geteilt ist, die aneinander zu klemmen sind.

6. System nach Anspruch 1, wobei der Adapter (50) ein elastischer Schlauch ist.

7. System nach Anspruch 6, wobei der elastische Schlauch über den Ultraschallkopf geschrumpft wird.

8. System nach Anspruch 1, wobei der Adapter (50) ein unelastischer Schlauchvorformling ist.

9. System nach Anspruch 8, wobei der Schlauchvorformling eine innenliegende Haftschicht aufweist.

10. System nach Anspruch 1, wobei die bildbasierte Volumenbewegung (VM_{IB}) als Funktion einer Bildposition (VLᵢᵢ) eines ersten Bildvolumens (13i) in dem Koordinatensystem (11) in Bezug auf eine Bildposition (VLⱼᵢ) eines zweiten Bildvolumens (13j) in dem Koordinatensystem (11) berechnet wird und/oder wobei die trackingbasierte Volumenbewegung (VM_{TB}) als Funktion einer getrackten Position (VLᵢₜ) eines ersten Bildvolumens (13i) in dem Koordinatensystem (11), wie sie durch die Trackingdaten (32) dargestellt wird, in Bezug auf eine getrackte Position (VLⱼₜ) eines zweiten Bildvolumens (13j) in dem Koordinatensystem (11), wie sie durch die Trackingdaten (32) dargestellt wird, berechnet wird.

11. System nach Anspruch 1, wobei die Berechnungsvorrichtung (40) so funktioniert, dass sie die Kalibrierungsmatrix durch die Lösung einer linearen Gleichung unter Verwendung der trackingbasierten Volumenbewegung und der bildbasierten Volumenbewegung berechnet.

12. System nach Anspruch 1, wobei der zumindest eine Lagesensor (30) ein elektromagnetischer Sensor und die Tracking-Vorrichtung (51) eine elektromagnetische Tracking-Vorrichtung ist.

13. Kalibrierungsverfahren basierend auf Volumenbewegung zum Betrieb eines ultraschallbildgeführten Systems, wobei das System Folgendes umfasst: Einen oder mehrere Ultraschallköpfe (20), die so funktionieren, dass sie Bildvolumen (13i, 13j) eines anatomischen Objekts (10) erzeugen, und einen Adapter (50) mit zumindest einem Lagesensor (30), wobei sich der zumindest eine Lagesensor (30) von einem Einsatzereignis zu einem anderen Einsatzereignis in einer veränderlichen Position in Bezug auf den einen oder mehrere Ultraschallköpfe (20) befindet, wobei das Verfahren die folgenden Schritte umfasst:
a) Erzeugen von Trackingdaten (32), die das Tracking des zumindest einen Lagesensors (30) in einem Koordinatensystem (11) darstellen,
b) Erzeugen von Bilddaten (22) des anatomischen Objekts (10) auf der Grundlage der Bildvolumen (13i, 13j), und
c) bei jedem Einsatzereignis Durchführen einer automatischen Selbstkalibrierung der Bilddaten (22) in Bezug auf das Koordinatensystem (11) des zumindest einen Lagesensors (30) durch die Berechnung einer Kalibrierungsmatrix (51) unter Verwendung einer bildbasierten Volumenbewegung (VM_{IB}) und einer trackingbasierten Volumenbewegung (VM_{TB}),
wobei die bildbasierte Volumenbewegung (VM_{IB}) eine Bildbewegung von zumindest zwei Bildvolumen (13i, 13j) in dem Koordinatensystem (11) darstellt, die von den Bilddaten (22) abgeleitet wird,
wobei die trackingbasierte Volumenbewegung (VM_{TB}) eine Trackingbewegung der Bildvolumen (13i, 13j) in dem Koordinatensystem (11) darstellt, die von den Trackingdaten (32) abgeleitet wird,
**dadurch gekennzeichnet, dass**
der Adapter (50) bei einem Einsatzereignis an einem der Ultraschallköpfe (20) befestigt werden kann und dass
das Verfahren ferner einen Schritt der Prüfung der aus der automatischen Selbstkalibrierung abgeleiteten Kalibrierungsmatrix (51) einschließlich einer Prüfung einer absoluten Differenz zwischen der bildbasierten Volumenbewegung (VM_{IB}) und der trackingbasierten Volumenbewegung (VM_{TB}) umfasst.

14. Computerprogramm mit Programmcodemitteln, die einen Computer veranlassen, die Schritte des Verfahrens nach Anspruch 13 auszuführen, wenn das genannte Computerprogramm auf dem Computer ausgeführt wird.

## Revendications

1. Système guidé par image ultrasonore, le système comprenant :
- une ou plusieurs sondes ultrasonores (20) servant à générer des volumes d'image (13i, 13j) d'un objet anatomique (10) ;
- un dispositif adaptateur (50) comprenant au moins un capteur de position (30) ;
- un dispositif de localisation (51) servant à générer des données de localisation (32) représentant une localisation du au moins un capteur de position (30) dans un système de coordonnées (11);
- un dispositif d'imagerie ultrasonore (21) servant à générer des données d'imagerie (22) de l'objet anatomique (10) sur la base des volumes d'image (13i, 13j) ; et
- un dispositif de calcul (40) servant à auto-étalonner automatiquement, pour chaque événement d'utilisation, les données d'imagerie (22) par rapport au système de coordonnées (11) du au moins un capteur de position (30) par le calcul d'une matrice d'étalonnage (51) au moyen d'un mouvement de volume basé sur l'image (VM_{IB}) et d'un mouvement de volume basé sur la localisation (VM_{TB}),
le mouvement de volume basé sur l'image (VM_{IB}) représentant un mouvement d'image d'au moins deux volumes d'image (13i, 13j) dérivant des données d'imagerie (22),
le mouvement de volume basé sur la localisation (VM_{TB}) représentant un mouvement de localisation des volumes d'image (13i, 13j) dérivant des données de localisation (32), **caractérisé en ce que**
le dispositif adaptateur (50) peut être attaché, pour un événement d'utilisation, à l'une des sondes ultrasonores (20), et **en ce que**
le dispositif de calcul (40) sert en outre à exécuter un test de validation de la matrice d'étalonnage (51) dérivant de l'auto-étalonnage automatique, comprenant un test d'un écart absolu entre le mouvement de volume basé sur l'image (VM_{IB}) et le mouvement de volume basé sur la localisation (VM_{TB}).

2. Système selon la revendication 1, dans lequel le système est non étalonné avant que le dispositif de calcul (40) auto-étalonne automatiquement les données d'imagerie (22) par rapport au système de coordonnées (11) du au moins un capteur de position (30).

3. Système selon la revendication 1, dans lequel le dispositif adaptateur (50) est réutilisable pour une pluralité d'événements d'utilisation.

4. Système selon la revendication 1, dans lequel le dispositif adaptateur (50) est une coque dure dans laquelle le au moins un capteur de position (30) est intégré ou à laquelle le au moins un capteur de position est attaché.

5. Système selon la revendication 4, dans lequel la coque dure est séparée en au moins deux parties pouvant être fixées les unes aux autres.

6. Système selon la revendication 1, dans lequel le dispositif adaptateur (50) est un tube élastique.

7. Système selon la revendication 6, dans lequel le tube élastique est thermorétracté sur la sonde ultrasonore.

8. Système selon la revendication 1, dans lequel le dispositif adaptateur (50) est un tube inélastique préformé.

9. Système selon la revendication 8, dans lequel le tube préformé comporte une couche adhésive interne.

10. Système selon la revendication 1, dans lequel le mouvement de volume basé sur l'image (VM_{IB}) est calculé en fonction d'un emplacement d'image (VLᵢᵢ) d'un premier volume d'image (13i) dans le système de coordonnées (11) par rapport à un emplacement d'image (VLⱼᵢ) d'un second volume d'image (13j) dans le système de coordonnées (11) et/ou dans lequel le mouvement de volume basé sur la localisation (VM_{TB}) est calculé en fonction d'un emplacement suivi (VLᵢₜ) d'un premier volume d'image (13i) dans le système de coordonnées (11) tel que représenté par les données de localisation (32) et d'un emplacement suivi (VLⱼₜ) d'un second volume d'image (13j) dans le système de coordonnées (11) tel que représenté par les données de localisation (32).

11. Système selon la revendication 1, dans lequel le dispositif de calcul (40) sert à calculer la matrice d'étalonnage par la résolution d'une équation linéaire à l'aide du mouvement de volume basé sur la localisation et du mouvement de volume basé sur l'image.

12. Système selon la revendication 1, dans lequel le au moins un capteur de position (30) est un capteur électromagnétique et le dispositif de localisation (51) est un dispositif de localisation électromagnétique.

13. Procédé d'étalonnage basé sur un mouvement de volume permettant de faire fonctionner un système guidé par image ultrasonore, le système comprenant une ou plusieurs sondes ultrasonores (20) servant à générer des volumes d'image (13i, 13j) d'un objet anatomique (10), et un dispositif adaptateur (50) comprenant au moins un capteur de position (30), dans lequel le au moins un capteur de position (30) se trouve à une position variable par rapport à la sonde ultrasonore (20) ou aux sondes ultrasonores (20) d'un événement d'utilisation à un autre événement d'utilisation, le procédé comprenant les étapes suivantes :
a) la génération de données de localisation (32) représentant une localisation du au moins un capteur de position (30) dans un système de coordonnées (11) ;
b) la génération de données d'imagerie (22) de l'objet anatomique (10) sur la base des volumes d'image (13i, 13j) ; et
c) l'auto-étalonnage automatique, pour chaque événement d'utilisation, des données d'imagerie (22) par rapport au système de coordonnées (11) du au moins un capteur de position (30) par le calcul d'une matrice d'étalonnage (51) au moyen d'un mouvement de volume basé sur l'image (VM_{IB}) et d'un mouvement de volume basé sur la localisation (VM_{TB}),
le mouvement de volume basé sur l'image (VM_{IB}) représentant un mouvement d'image d'au moins deux volumes d'image (13i, 13j) dans le système de coordonnées (11) dérivant des données d'imagerie (22),
le mouvement de volume basé sur la localisation (VM_{TB}) représentant un mouvement de localisation des volumes d'image (13i, 13j) dans le système de coordonnées (11) dérivant des données de localisation (32),
**caractérisé en ce que**
le dispositif adaptateur (50) peut être attaché, pour un événement d'utilisation, à l'une des sondes ultrasonores (20), et **en ce que**
le procédé comprend en outre une étape de test de la matrice d'étalonnage (51) dérivant de l'auto-étalonnage automatique, comprenant un test d'un écart absolu entre le mouvement de volume basé sur l'image (VM_{IB}) et le mouvement de volume basé sur la localisation (VM_{TB}).

14. Programme informatique, comprenant un moyen de code de programme permettant d'amener un ordinateur à mettre en oeuvre les étapes du procédé selon la revendication 13 quand ledit programme informatique est exécuté sur l'ordinateur.
